(19) Europäisches Patentamt
European Patent Office
Office européen des brevets

(11) Numéro de publication : **0 330 193 B1**

# (12) FASCICULE DE BREVET EUROPEEN

(45) Date de publication du fascicule du brevet : **23.09.92 Bulletin 92/39**

(51) Int. Cl.$^5$ : **A61K 7/06,** A61K 7/00, A61K 7/42

(21) Numéro de dépôt : **89103151.0**

(22) Date de dépôt : **23.02.89**

(54) **Composition cosmétique pour le maintien de la coiffure contenant un polymère d'oxazoline et un acide 2-hydroxy 4-methoxy benzophénone 5-sulfonique salifié ou non.**

Le dossier contient des informations techniques présentées postérieurement au dépôt de la demande et ne figurant pas dans leprésent fascicule.

(30) Priorité : **25.02.88 LU 87142**

(43) Date de publication de la demande : **30.08.89 Bulletin 89/35**

(45) Mention de la délivrance du brevet : **23.09.92 Bulletin 92/39**

(84) Etats contractants désignés : **AT BE CH DE FR GB GR IT LI NL SE**

(56) Documents cités : **EP-A- 0 193 932**

(56) Documents cités :
**DE-A- 1 939 669**
**DE-A- 2 046 818**
**FR-A- 1 553 988**
**GB-A- 671 857**

(73) Titulaire : **L'OREAL**
**14, Rue Royale**
**F-75008 Paris (FR)**

(72) Inventeur : **Dupuis, Christine**
**15, Rue Seveste**
**F-75018 Paris (FR)**
Inventeur : **Grollier, Jean-François**
**16 bis Boulevard Morland**
**F-75004 Paris (FR)**

(74) Mandataire : **Casalonga, Axel et al**
**BUREAU D.A. CASALONGA - JOSSE**
**Morassistrasse 8**
**W-8000 München 5 (DE)**

EP 0 330 193 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet une nouvelle composition cosmétique destinée à être utilisée pour le maintien des cheveux, contenant un polymère d'oxazoline et un acide 2-hydroxy 4-méthoxy benzophénone 5-sulfonique salifié ou non.

Les polymères dérivés d'oxazoline sont connus en eux-mêmes et le brevet français 1.553.988 décrit notamment l'application de certains de ces polymères dans des compositions pour la coiffure destinées à maintenir les cheveux en place, qu' il s'agisse de maintenir la mise en plis, l'ondulation permanente ou de fixer la coiffure.

Ces compositions s'appliquent directement sur les cheveux à l'aide d'un peigne ou à la main et sont de préférence appliquées par pulvérisation sur la chevelure, auquel cas elles constituent des laques.

Les polymères d'oxazoline sont parfaitement adaptés pour ce type d'application qui requiert une bonne solubilité dans les solvants organiques tels que l'éthanol et l'isopropanol, une compatibilité élevée avec les propulseurs ainsi qu'une dispersibilité aisée dans l'eau afin de pouvoir s'éliminer rapidement des cheveux par rinçage ou par les shampooings.

En outre, ces résines ne requièrent pas obligatoirement l'emploi d'additifs tels que des plastifiants car elles forment elles-mêmes des pellicules flexibles.

Par ailleurs, les pellicules formées avec ces polymères sont brillantes et conservent parfaitement bien l'ondulation.

La demanderesse a cependant constaté que dans certains cas les polymères d'oxazoline réagissaient avec le sébum du cheveu, secrété naturellement par les glandes sébacées, en produisant un effet collant désagréable au toucher et un aspect inesthétique de la chevelure.

La demanderesse a découvert de façon surprenante que l'addition d'acide 2-hydroxy 4-méthoxy benzophénone 5-sulfonique salifié ou non, aux polymères d'oxazoline, permettait de remédier à cet inconvénient d'effet collant sur les cheveux sans toutefois modifier les propriétés avantageuses conférées par lesdits polymères.

L'invention a donc pour objet une composition cosmétique destinée au maintien des cheveux contenant au moins un polymère d'oxazoline et au moins un acide 2-hydroxy 4-méthoxy benzophénone 5-sulfonique salifié ou non.

Un autre objet de l'invention est constitué par un procédé de traitement cosmétique des cheveux destiné à favoriser leur maintien consistant à leur appliquer l'association d'au moins un polymère d'oxazoline et d'au moins un acide 2-hydroxy 4-méthoxy benzophénone 5-sulfonique salifié ou non.

D' autres objets de l'invention apparaîtront à la lecture de la description et des exemples qui suivent.

La composition cosmétique conforme à l'invention est essentiellement caractérisée par le fait qu'elle contient dans un milieu cosmétiquement acceptable au moins un polymère d'oxazoline de formule (I) :

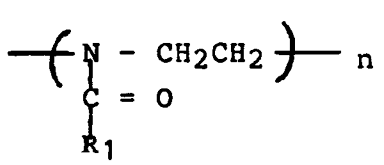

(I)

dans laquelle $R_1$ est un radical alkyle inférieur en $C_1$-$C_4$, et de préférence un groupement éthyle, n a une valeur telle que le poids moléculaire est au moins égal à 10.000, et au moins un acide 2-hydroxy 4-méthoxy benzophénone 5-sulfonique ou ses sels cosmétiquement acceptables.

Les sels cosmétiquement acceptables sont choisis parmi les sels métalliques tels que plus particulièrement les sels alcalins et alcalino-terreux, les sels d'ammonium et d'amines.

Les polymères d'oxazoline de formule (I) ont un poids moléculaire supérieur à 10.000, généralement compris entre 20.000 et 1.000.000, et de préférence entre 50.000 et 500.000 et sont préparés par polymérisation de 2-alkyl 2-oxazoline. Les polymères préférés sont les homopolymères d'éthyloxazoline ayant un poids moléculaire compris entre 20.000 et 1.000.000, et plus particulièrement ceux vendus sous la dénomination de PEOX par la Société DOW CHEMICAL ayant des poids moléculaires de 50.000 à 500.000.

Un polymère plus particulièrement préféré selon l'invention est représenté par un homopolymère d'éthyloxazoline de poids moléculaire 50.000.

L'acide 2-hydroxy 4-méthoxy benzophénone 5-sulfonique utilisé conformément à l'invention est de préférence celui vendu sous la dénomination "UVINUL MS 40" par la Société BASF.

Le polymère d'oxazoline est présent dans les compositions conformes à l'invention, dans des concentrations comprises entre O,2 et 1O% en poids par rapport au poids total de la composition, de préférence de O,5 à 8%, et en particulier comprises entre 1,3 et 5% en poids, et l'acide 2-hydroxy 4-méthoxy benzophénone 5-sulfonique est utilisé dans des concentrations comprises de préférence entre O,O5 et 2,5% en poids, et plus particulièrement entre O,1 et 2% en poids, par rapport au poids total de la composition.

Les compositions conformes à l'invention peuvent se présenter sous forme de lotions aqueuses, hydroalcooliques ou alcooliques, éventuellement épaissies, et de préférence être conditionnées en aérosol pour être appliquées sous forme de pulvérisation ou spray et former une laque sur les cheveux.

Le milieu cosmétiquement acceptable peut être constitué par de l'eau et/ou un solvant organique cosmétiquement acceptable tel que plus particulièrement un solvant choisi parmi les monoalcools ayant de 1 à 8 atomes de carbone comme l'éthanol, l'isopropanol, l'alcool benzylique, l'alcool phényléthylique, les polyalcools tels que les alkylèneglycols comme l'éthylèneglycol, le propylèneglycol, utilisés seuls ou en mélanges. Le solvant est présent de préférence dans des proportions inférieures ou égales à 7O% en poids par rapport au poids total de la composition totale.

Lorsque les compositions se présentent sous forme de lotions hydroalcooliques, la quantité d'alcool présente dans la composition peut être de 5 à 7O% en poids par rapport au poids total de la composition.

Lorsque les compositions selon l'invention sont conditionnées en aérosol pour être appliquées sous forme de spray, elles sont essentiellement alcooliques et contiennent une quantité d'alcool et notamment d'alcool éthylique, dans une proportion de 2O à 95% et plus particulièrement de 2O à 7O% en poids par rapport au poids total de la composition pressurisée.

Les compositions conformes à la présente invention peuvent contenir des additifs habituellement utilisés en cosmétique, compatibles avec les polymères d'oxazoline de formule (I), choisis parmi les émollients, les lubrifiants, les agents pénétrants, les stabilisants, les parfums, les agents épaississants, les agents conservateurs et éventuellement les plastifiants.

Les épaississants, utilisés dans les compositions conformes à l'invention, sont choisis de préférence parmi les polymères d'acide acrylique réticulés ou non, et plus particulièrement les acides polyacryliques réticulés par un agent polyfonctionnel tels que les produits vendus sous la dénomination CARBOPOL® par la Société GOODRICH, les dérivés de cellulose tels que la méthylcellulose, l'hydroxyéthylcellulose, l'hydroxypropylméthylcellulose, les sels de sodium de la carboxyméthylcellulose ou les copolymères éthylène/anhydride maléique de haut poids moléculaire.

Ces épaississants sont présents entre O,O5 et 5%, et de préférence entre O,1 et 2% en poids par rapport au poids total de la composition.

Lorsque les compositions conformes à l'invention sont conditionnées en aérosol pour être appliquées sous forme de spray, les agents propulseurs peuvent être choisis parmi les hydrocarbures volatils tels que le n-butane, le propane, l'isobutane ou leurs mélanges ou un mélange de ces hydrocarbures avec des hydrocarbures chlorés et/ou fluorés tels que les composés vendus sous la dénomination de Fréon® ou Dymel® par la Société DU PONT DE NEMOURS et plus particulièrement les hydrocarbures fluorochlorés tels que le monofluorotrichlorométhane, le difluorodichlorométhane, le tétrafluorodichloroéthane ou les mélanges de ces derniers.

Ils peuvent également être choisis parmi les hydrocarbures chlorés et/ou fluorés susdécrits et leurs mélanges, l'éther diméthylique, le gaz carbonique et le protoxyde d'azote.

On préfère, selon l'invention, utiliser un mélange de n-butane, isobutane, propane et de monofluorotrichlorométhane et plus particulièrement un mélange de n-butane, d'isobutane et de propane.

La phase propulsive, dans ces compositions de laque, représente 3O à 8O% du poids total de la composition pressurisée.

Les compositions conformes à l'invention sont utilisées pour la fixation des cheveux. Elles peuvent être appliquées comme produit de traitement après la coloration, décoloration, après le shampooing, après la permanente ou le défrisage des cheveux.

Une composition particulièrement préférée selon l'invention est constituée par une composition pour fixer les cheveux sous forme de spray ou de laque.

Le procédé de traitement cosmétique des cheveux destiné à favoriser leur maintien qui constitue un autre objet de l'invention consiste à appliquer au moins un polymère d'oxazoline de formule (I) et au moins un acide 2-hydroxy 4-méthoxy benzophénone 5-sulfonique salifié ou non, dans un milieu cosmétiquement acceptable, sur les cheveux mouillés ou séchés, au moyen des compositions décrites ci-dessus, de préférence sans faire suivre l'application d'un rinçage.

Les exemples suivants sont destinés à illustrer l'invention sans pour autant présenter un caractère limitatif.

EXEMPLE 1

On prépare un spray de composition suivante :

| | | |
|---|---|---|
| - Polyéthyloxazoline de PM 50.000, vendu sous la dénomination PEOX® par la Société DOW CHEMICAL | | 5,83 g |
| - Acide 2-hydroxy 4-méthoxy benzophénone 5-sulfonique (UVINUL® MS 40 de BASF) | | 0,83 g |
| - Parfum | qs | |
| - Alcool éthylique absolu | qsp | 100,00 g |

Conditionnement aérosol :

| | | |
|---|---|---|
| - Composition ci-dessus | | 48,00 g |
| - Mélange ternaire de n-butane, isobutane > 55%, propane, vendu sous la dénomination AEROGAZ® 3,2 N par la Société ELF AQUITAINE | | 52,00 g |
| | TOTAL | 100,00 g |

EXEMPLE 2

On prépare un spray de composition suivante :

| | | |
|---|---|---|
| - Polyéthyloxazoline de PM 50.000, vendu sous la dénomination PEOX par la Société DOW CHEMICAL | | 2,72 g |
| - Acide 2-hydroxy 4-méthoxy benzophénone 5-sulfonique (UVINUL MS 40 de BASF) | | 0,63 g |
| - Parfum | qs | |
| - Alcool éthylique absolu | qsp | 100,00 g |

Conditionnement aérosol :

| | | |
|---|---|---|
| - Composition ci-dessus | | 55,00 g |
| - Mélange ternaire de n-butane, isobutane > 55%, propane, vendu sous la dénomination AEROGAZ 3,2 N par la Société ELF AQUITAINE | | 45,00 g |
| | TOTAL | 100,00 g |

EXEMPLE 3

On prépare un gel de coiffage de composition suivante :

| | | |
|---|---|---|
| - Acide polyacrylique réticulé PM : 4.000.000, vendu sous la dénomination CARBOPOL 940 par la Société GOODRICH | | 1,20 g |
| - Polyéthyloxazoline de PM : 500.000, vendu sous la dénomination PEOX par la Société DOW CHEMICAL | | 3,00 g |
| - Acide 2-hydroxy 4-méthoxy benzophénone 5-sulfonique (UVINUL MS 40 de BASF) | | 0,50 g |
| - Sel pentasodique de l'acide diéthylène triaminopentacétique | | 0,20 g |
| - Alcool éthylique | | 17,20 g |
| - Triéthanolamine qs pH = 7 | | |
| - Parfum, colorant, qs | | |
| - Eau | qsp | 100,00 g |

## Revendications

### Revendications pour les Etats contractants suivants : AT,BE,CH,DE,FR,GB,GR,IT,LI,NL,SE

1. Composition cosmétique destinée au maintien des cheveux, caractérisée par le fait qu'elle contient dans un milieu cosmétiquement acceptable au moins un polymère d'oxazoline de formule (I) :

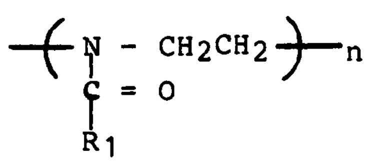

(I)

dans laquelle $R_1$ est un radical alkyle inférieur en $C_1$-$C_4$, n a une valeur telle que le poids moléculaire est au moins égal à 10.000 et au moins un acide 2-hydroxy 4-méthoxy benzophénone 5-sulfonique ou ses sels cosmétiquement acceptables.

2. Composition cosmétique selon la revendication 1, caractérisée par le fait que les polymères d'oxazoline de formule (I) ont un poids moléculaire de 20.000 à 1.000.000.

3. Composition selon l'une des revendications 1 et 2, caractérisée par le fait que le polymère d'oxazoline de formule (I) est un homopolymère d'éthyloxazoline de poids moléculaire 50.000.

4. Composition selon l'une des revendications 1 à 3, caractérisée par le fait qu'elle contient au moins de 0,2 à 10% de polymère d'oxazoline de formule (I) en poids par rapport au poids total de la composition et au moins de 0,05 à 2,5% en poids d'acide 2-hydroxy 4-méthoxy benzophénone 5-sulfonique salifié ou non.

5. Composition selon la revendication 4, caractérisée par le fait qu'elle contient dans un milieu cosmétiquement acceptable :

– au moins de 1,3 à 5% en poids de polymère d'oxazoline de formule (I);

– au moins de 0,1 à 2% en poids d'acide 2-hydroxy 4-méthoxy benzophénone 5-sulfonique salifié ou non.

6. Composition selon l'une des revendications 1 à 5, caractérisée par le fait qu'elle se présente sous forme de lotion aqueuse, hydroalcoolique ou alcoolique, épaissie ou non.

7. Composition selon l'une des revendications 1 à 6, caractérisée par le fait qu'elle est conditionnée en aérosol pour être appliquée sous forme de pulvérisation ou de spray et former une laque sur les cheveux.

8. Composition selon l'une des revendications 1 à 7, caractérisée par le fait que le milieu cosmétiquement acceptable contient de l'eau et/ou un solvant organique cosmétiquement acceptable choisi parmi les monoalcools ayant de 1 à 8 atomes de carbone, les polyalcools utilisés seuls ou en mélanges, ledit solvant étant présent dans des proportions inférieures ou égales à 7O% en poids par rapport au poids total de la composition.

9. Composition sous forme de lotion hydroalcoolique selon la revendication 8, caractérisée par le fait que la quantité d'alcool présent dans la composition est comprise entre 5 et 7O% en poids par rapport au poids total de la composition.

1O. Composition conditionnée en aérosol sous forme de laque selon l'une quelconque des revendications 1 à 7, caractérisée par le fait q'elle contient de 2O à 95% d'alcool en poids par rapport au poids total de la composition pressurisée.

11. Composition selon l'une quelconque des revendications 1 à 1O, caractérisée par le fait qu'elle contient des additifs compatibles avec le polymère d'oxazoline de formule (I) choisis parmi des émollients, des parfums, des agents conservateurs, des agents épaississants, des lubrifiants, des plastifiants ou des stabilisants.

12. Composition selon l'une quelconque des revendications 1 à 11, caractérisée par le fait qu'elle contient de O,O5 à 5% en poids et de préférence de O,1 à 2% en poids par rapport au poids total de la composition d'un agent épaississant choisi parmi les polymères d'acide acrylique réticulés ou non, les dérivés de cellulose ou les copolymères éthylène/anhydride maléique de haut poids moléculaire.

13. Composition selon l'une des revendications 1 à 12, conditionnée en aérosol pour être appliquée sous forme de spray, caractérisée par le fait qu'elle contient dans une proportion de 3O à 8O% en poids par rapport au poids total de la composition pressurisée, un agent propulseur choisi parmi le n-butane, le propane, l'isobutane ou leurs mélanges ou un mélange de ces hydrocarbures avec des hydrocarbures chlorés et/ou fluorés choisis parmi le monofluorotrichlorométhane, le difluorodichlorométhane, le tétrafluorodichloroéthane, des mélanges de ces hydrocarbures fluorés et/ou chlorés susdécrits, le gaz carbonique, l'éther diméthylique ou le protoxyde d'azote.

14. Composition selon la revendication 13, caractérisée par le fait qu'elle contient un agent propulseur choisi parmi un mélange de n-butane, d'isobutane, de propane et de monofluorotrichlorométhane ou un mélange de n-butane, propane et d'isobutane.

15. Utilisation de la composition telle que définie dans l'une des revendications 1 à 14, pour la fixation des cheveux, comme produit de traitement pouvant être appliqué après la coloration, décoloration, après le shampooing, après la permanente ou le défrisage des cheveux.

16. Procédé de traitement cosmétique des cheveux destiné à favoriser leur maintien, caractérisé par le fait que l'on applique au moins un polymère d'oxazoline de formule (I) et au moins un acide 2-hydroxy 4-méthoxy benzophénone 5-sulfonique salifié ou non tels que définis dans la revendication 1, dans un milieu cosmétiquement acceptable, sur les cheveux mouillés ou séchés, cette application n'étant pas suivie d'un rinçage.

## Patentansprüche

### Patentanspruch für folgende Vertragsstaaten : AT, BE, CH, DE, FR, GB, GR, IT, LI, NL, SE

I. Kosmetische Zusammensetzung zur Erhaltung der Frisur, dadurch **gekennzeichnet**, daß sie in einem kosmetisch verträglichen Milieu mindestens ein Oxazolinpolymer der Formel (I):

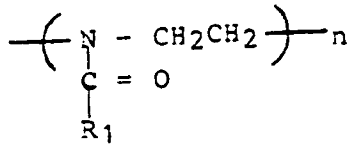

(I)

worin $R_l$ ein $C_l$-$C_4$-Niedrigalkylrest ist und n einen Wert aufweist, so daß das Molekulargewicht mindestens l0.000 beträgt, sowie mindestens eine 2-Hydroxy-4-methoxybenzophenon-5-sulfonsäure oder deren kosmetisch verträglichen Salze enthält.

2. Kosmetische Zusammensetzung gemäß Anspruch I,
dadurch **gekennzeichnet**, daß
die Oxazolinpolymeren der Formel (I) ein Molekulargewicht von 20.000 bis I00.000 aufweisen.

3. Zusammensetzung gemäß der Ansprüche I und 2,

dadurch **gekennzeichnet**, daß
das Oxazolinpolymer der Formel (I) ein Homopolymer von Ethyloxazolin mit Molekulargewicht 50.000 ist.

4. Zusammensetzung gemäß eines der Ansprüche I bis 3,
dadurch **gekennzeichnet**, daß
sie mindestens 0,2 bis l0 Gew.% Oxazolinpolymer der Formel (I), bezogen auf Gesamtgewicht der Zusammensetzung, und mindestens 0,05 bis 2,5 Gew.% 2-Hydroxy-4-methoxybenzophenon-5-sulfonsäure oder deren Salze enthält.

5. Zusammensetzung gemäß Anspruch 4,
dadurch **gekennzeichnet**, daß
sie in einem kosmetisch verträglichen Milieu enthält:
– mindestens I,3 bis 5 Gew.% Oxazolinpolymer der Formel (I);
– mindestens 0,l bis 2 Gew.%
2-Hydroxy-4-methoxybenzophenon-5-sulfonsäure oder deren Salze.

6. Zusammensetzung gemäß eines der Ansprüche I bis 5,
dadurch **gekennzeichnet**, daß
sie in Form einer wässrigen, hydroalkoholischen oder alkoholischen Lotion, verdickt oder nicht, vorliegt.

7. Zusammensetzung gemäß eines der Ansprüche I bis 6,
dadurch **gekennzeichnet**, daß
sie als Aerosol zubereitet ist, um in pulverisierter Form oder als Spray aufgebracht zu werden und auf den Haaren einen Lack zu bilden.

8. Zusammensetzung gemäß eines der Ansprüche I bis 7,
dadurch **gekennzeichnet**, daß
das kosmetisch verträgliche Milieu Wasser und/oder ein kosmetisch verträgliches organisches Lösungsmittel enthält, ausgewählt aus Monoalkoholen mit I bis 8 Kohlenstoffatmen und Polyalkoholen, alleine oder in Mischungen, wobei das genannte Lösungsmittel in Mengen unterhalb oder gleich 70 Gew.%, bezogen auf Gesamtgewicht der Zusammensetzung, vorliegt.

9. Zusammensetzung in Form einer hydroalkoholischen Lotion gemäß Anspruch 8,
dadurch **gekennzeichnet**, daß
die in der Zusammensetzung vorliegende Alkoholmenge 5 bis 70 Gew.%, bezogen auf Gesamtgewicht der Zusammensetzung, beträgt.

I0. Zusammensetzung als Aerosolzubereitung in Form eines Lackes gemäß eines jeden der Ansprüche I bis 7,
dadurch **gekennzeichnet**, daß
sie 20 bis 95 Gew.% Alkohol enthält, bezogen auf Gesamtgewicht der unter Druck gesetzten Zusammensetzung.

II. Zusammensetzung gemäß eines jeden der Ansprüche I bis I0,
dadurch **gekennzeichnet**, daß
sie mit dem Oxazolinpolymer der Formel (I) kompatible Additive enthält, ausgewählt aus Weichmachern, Parfum, Konservierungs-, Verdickungs-, Gleit-, Plastifizier- oder Stabilisiermitteln.

I2. Zusammensetzung gemäß eines der Ansprüche I bis II,
dadurch **gekennzeichnet**, daß
sie 0,05 bis 5 Gew.%, vorzugsweise 0,l bis 2 Gew.%, bezogen auf Gesamtgewicht der Zusammensetzung, eines Verdickungsmittels enthält, ausgewählt aus Acrylsäurepolymeren, vernetzt oder nicht, Cellulosederivaten oder Ethylen/Maleinsäureanhydrid-Copolymeren mit hohem Molekulargewicht.

I3. Zusammensetzung gemäß eines der Ansprüche I bis I2, zubereitet als Aerosol zur Aufbringung in Form eines Sprays,
dadurch **gekennzeichnet**, daß
sie in einer Menge von 30 bis 80 Gew.%, bezogen auf Gesamtgewicht der unter Druck gesetzten Zusammensetzung, eines Treibmittels enthält, ausgewählt aus n-Butan, propan, Isobutan oder deren Mischungen oder aus einer Mischung dieser Kohlenwasserstoffe mit chlorierten und/oder fluorierten Kohlenwasserstoffen, ausgewählt aus Monofluortrichlormethan, Difluordichlormethan, Tetrafluordichlorethan, Mischungen dieser fluorierten und/oder chlorierten Kohlenwasserstoffe, Kohlendioxid, Dimethylether oder Distickstoffmonoxid (Stickstoffprotoxid).

I4. Zusammensetzung gemäß Anspruch I3,
dadurch **gekennzeichnet**, daß
sie ein Treibmittel enthält, ausgewählt aus einer Mischung von n-Butan, Isobutan, propan und Monofluortrichlormethan oder einer Mischung aus n-Butan, propan und Isobutan.

I5. Verwendung der Zusammensetzung gemäß eines der Ansprüche I bis I4 als Behandlungsmittel zur Fi-

xierung der Haare, um nach der Färbung, Entfärbung, dem Shampoonieren, nach der Dauerwelle oder dem Auskämmen der Haare aufgebracht werden zu können.

I6. Verfahren zur kosmetischen Behandlung der Frisur, um deren Erhaltung zu begünstigen, dadurch **gekennzeichnet**, daß man mindestens ein Oxazolinpolymer der Formel (I) und mindestens eine 2-Hydroxy-4-methoxybenzophenon-5-sulfonsäure oder deren Salze, wie in Anspruch I definiert, in einem kosmetisch verträglichen Milieu auf die feuchten oder trockenen Haare aufbringt, wobei man dieser Aufbringung keine Spülung folgen läßt.

## Claims

1. Cosmetic composition designed to maintain hair in place, characterized in that it contains, in a cosmetically acceptable medium, at least one oxazoline polymer of formula (I) :

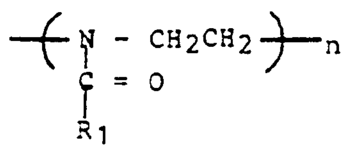

in which $R_1$ is a $C_1$-$C_4$ lower alkyl radical and n has a value such that the molecular weight is equal to at least 10,000, and a least one 2-hydroxy-4-methoxybenzophenone-5-sulphonic acid or its cosmetically acceptable salts.

2. Cosmetic composition according to Claim 1, characterized in that the oxazoline polymers of formula (I) have a molecular weight of 20,000 to 1,000,000.

3. Composition according to one of Claims 1 and 2, characterized in that the oxazoline polymer of formula (I) is a homopolymer of ethyloxazoline of molecular weight 50,000.

4. Composition according to one of Claims 1 to 3, characterized in that it contains at least 0.2 to 10% of oxazoline polymer of formula (I) by weight relative to the total weight of the composition, and at least 0.05 to 2.5% by weight of 2-hydroxy-4-methoxybenzophenone-5-sulphonic acide, salified or otherwise.

5. Composition according to Claim 4, characterized in that it contains, in a cosmetically acceptable medium :
at least 1.3 to 5% by weight of oxazoline polymer of formula (I); and
at least 0.1 to 2% by weight of 2-hydroxy-4-methoxybenzophenone 5-sulphonic acid, salified or otherwise.

6. Composition according to Claims 1 to 5 , characterized in that it is presented in the form of an aqueous, aqueous-alcoholic or alcoholic lotion, thickened or otherwise.

7. Composition according to one of Claims 1 to 6 , characterized in that it is packaged as an aerosol for application in the form of an atomization or spray and to form a lacquer on the hair.

8. Composition according to one of Claims 1 to 7, characterized in that the cosmetically acceptable medium contains water and/or a cosmetically acceptable organic solvent, chosen from monohydric alcohols having from 1 to 8 carbon atoms and polyhydric alcohols, used alone or as mixtures, said solvent being present in proportions of not more than 70% by weight relative to the total weight of the composition.

9. Composition in the form of an aqueous-alcoholic lotion according to Claim 8, characterized in that the quantity of alcohol present in the composition is between 5 and 70% by weight relative to the total weight of the composition.

10. Composition packaged as an aerosol in the form of a lacquer according to any one of Claims 1 to 7, characterized in that it contains from 20 to 95% of alcohol by weight relative to the total weight of the pressurized composition.

11. Composition according to any one of Claims 1 to 10, characterized in that it contains additives which are

compatible with the oxazoline polymer of formula (I) and chosen from emollients,perfumes, preservatives, thickening agents, lubricants, plasticizers or stabilizers.

**12.** Composition according to any one of Claims 1 to 11, characterized in that it contains from 0.05 to 5% by weight, and preferably from 0.1 to 2% by weight , relative to the total weight of the composition, of a thickening agent chosen from acrylic acid polymers, cross linked or otherwise, cellulose derivatives or high molecular weight ethylene/maleic anhydride copolymers.

**13.** Composition according to one of Claims 1 to 12, packaged as an aerosol for application in spray form, characterized in that it contains, in a proportion of 30 to 80% by weight relative to the total weight of the pressurized composition, a propellant agent chosen from n-butane, propane and isobutane or mixtures thereof , or a mixture of these hydrocarbons with chlorinated and/or fluorinated hydrocarbons chosen from monofluorotrichloromethane,difluorodichloromethane, and tetrafluorodichloroethane, mixtures of these fluorinated and/or chlorinated hydrocarbons described above, carbon dioxide, dimethyl ether or nitrous oxide.

**14.** Composition according to Claim 13, characterized in that it contains a propellant agent chosen from a mixture of n-butane, isobutane, propane and monofluorotrichloromethane, or a mixture of n-butane, propane and isobutane.

**15.** Use of the composition as defined in one of Claims 1 to 14, fo fixing the hair in place , as a treatment product which can be applied after dyeing or bleaching, after shampooing and permanent waving or straightening of the hair.

**16.** Process for the cosmetic treatment of the hair designed to promote maintenance of the latter in place, characterized in that at least one oxazoline polymer of formula (I) and at least one 2-hydroxy-4-methoxybenzophenone-5-sulphonic acid, salified or otherwise, as defined in Claim 1, are applied in a cosmetically acceptable medium on the wet or dried hair, this application not being followed by rinsing.